# EUROPEAN PATENT APPLICATION

(11) **EP 2 301 558 A1**
(43) Date of publication of application: **30.03.2011**
(21) Application number: 09750682.8
(22) Date of filing: 19.05.2009
(51) Int. Cl.: A61K 36/18, A23K 1/16, A23L 1/30, A61K 36/00, A61P 17/00, A61P 17/04, A61P 37/08

(54) **PREVENTATIVE AND/OR THERAPEUTIC AGENT AGAINST ATOPIC DERMATITIS**

(30) Priority: 22.05.2008 JP 2008134246
(71) Applicant: Lotte Co., Ltd., Tokyo 160-0023 (JP)
(72) Inventor: KURODA, Reiko, Saitama-shi Saitama 336-0027 (JP); HIGUCHI, Hiroaki, Saitama-shi Saitama 336-0027 (JP); NARISE, Atsushi, Saitama-shi Saitama 336-0027 (JP); SHIMIZU, Katsumasa, Saitama-shi Saitama 336-0027 (JP); OSAWA, Kenji, Saitama-shi Saitama 336-0027 (JP)
(74) Representative: Hartz, Nikolai
(86) International application number: PCT/JP2009/059492
(87) International publication number: WO 2009/142320

(57) **Abstract**

To provide a prophylactic and/or therapeutic agent for atopic dermatitis with minimal concerns of adverse drug reactions and high safety, various researches were conducted. As a result, it was found that an extract extracted from a mangosteen (Garcinia mangostana L.) peel with a polar solvent prevented or healed atopic dermatitis. That is, the present invention provides a prophylactic and/or therapeutic agent for atopic dermatitis comprising an extract extracted from a mangosteen peel with a polar solvent. Furthermore, the present invention provides a food containing the above-mentioned prophylactic and/or therapeutic agent for atopic dermatitis.

## Description

### TECHNICAL FIELD

The present invention relates to a prophylactic and/or therapeutic agent for atopic dermatitis comprising an extract of a mangosteen peel.

### BACKGROUND ART

Atopic dermatitis is a disease that presents with repeated exacerbation and remission of eczema associated with pruritus as the main lesion. Many patients are thought to have an atopic disposition. The atopic disposition refers to (1) a family history or past medical history or (2) a predisposition of producing an IgE antibody.

The main lesions of atopic dermatitis include skin erythema or papule, cuts behind ears, dry skin, follicular keratotic papule associated with pityriasis, and scratch scars in the affected skin area. According to a recently conducted survey, the prevalence of atopic dermatitis is 12.8% among four-month-old infants, 9.8% among 18-month-old infants, 13.2% among three-year-old children, 11.8% among the first-grade primary school children, 10.6% among the sixth-grade primary school children, and 8.2% among the first-year university students. The prevalence is high among children, with one in ten children. It is thought that the major causative and exacerbating factors include food, diaphoresis, environmental factors, bacteria, fungi, contact antigens, and stress.

Atopic dermatitis is treated by 1) search of and countermeasures against causative and exacerbating factors, 2) skin care, and 3) a drug therapy. If symptoms are not relieved by 1) or 2), a drug therapy is performed. As the drug, topical steroid agents are most widely used, and many types of topical steroid agents are available. These agents are classified into five ranks from weak to strongest depending on the clinical effect and selected for use depending on the severity of the condition or the patient's age. Meanwhile, usefulness of Protopic, an immunomodifier, which is a topical non-steroid agent, has been recognized in recent years. Furthermore, antihistamines, antiallergic agents, and the like are used as oral agents, and internal steroid medicines may be temporarily used in patients with the most severe condition.

However, when a steroid is topically used, adverse drug reactions such as skin atrophy, vascular dilatation, and folliculitis may develop. The guideline prepared by a scientific research group of the Health, Labour and Welfare Ministry of Japan instructs not to use a topical steroid on the face wherever possible. Furthermore, many patients are concerned about adverse drug reactions of steroids and negatively respond to their use. Protopic is a relatively new drug which was approved for its use in November 1999, and only use at low concentrations has been approved in patients under 16 years of age. Furthermore, even use at low concentrations has not been approved in children under two years of age. Antihistamines and antiallergic agents that are internally administered may cause adverse drug reactions such as sleepiness, lassitude, and difficulty in coughing up of sputum caused by an anticholinergic action.

Therefore, to develop a drug that causes minimal adverse drug reactions and is readily available, researches have been conducted to explore extracts from natural products that can be used as prophylactic or therapeutic agents for atopic dermatitis.

Furthermore, atopic dermatitis is known to develop in animals other than humans, such as dogs, cats, and livestock, and prophylactic and therapeutic treatments for them are required.

Peels of mangosteen (Garcinia mangostana L.), a plant belonging to the family Guttiferae, are used as a folk remedy in Thailand to stop diarrhea or treat inflammation. In recent years, it has been reported that α-mangosteen and γ-mangosteen contained in mangosteen peels suppress allergic reactions.

Japanese Patent No. 3968405 discusses antihistamine effects or antiserotonin effects of α-mangosteen and γ-mangosteen purified from a mangosteen extract and effects of suppressing human pollinosis exhibited by α-mangosteen and γ-mangosteen. Japanese Patent Application Laid-Open No. 2005-298379 discusses effects of inhibiting IκB kinase exhibited by mangosteen extracts, in particular, α-mangosteen and γ-mangosteen. Japanese Patent Application Laid-Open No. 2001-278770 discusses effects of relieving effects of stress exhibited by extracts from plants belonging to the family Guttiferae.

Furthermore, mechanisms of antiallergic effects of mangosteen extracts have also been studied. Biol. Pharm. Bull., 25, p.1137 (2002) discusses inhibition of histamine release and prostaglandin E synthesis by mangosteen extracts. Eur. J. Pharmacol., 314, p.351 (1996) discusses α-mangosteen being an antagonist of the histamine H receptor. Mol. Pharmacol., 66, p.667 (2004) discusses inhibition of the IκB kinase activity and the release of prostaglandin E by γ-mangosteen and the resulting suppression of the cyclooxygenase 2 gene expression. Biochem. Pharmacol., 63, p.73 (2002) discusses suppression of syntheses of cyclooxygenase and prostaglandin E2 by γ-mangosteen.

Various studies have been conducted to examine safety of mangosteen peel extracts. Paragraph 0015 in Japanese Patent Application Laid-Open No. H5-17365 describes an acute toxicity study of oral administration in mice in which no death occurred after administration of 10 g/kg of a mangosteen peel extract. Furthermore, Paragraph 0047 in Japanese Patent Application Laid-Open No. H4-244004 describes an irritation test performed in a safety study showing that a mangosteen peel extract applied to the human skin has a very low irritating property and high safety.

### DISCLOSURE OF THE INVENTION

Japanese Patent No. 3968405 and Japanese Patent Application Laid-Open No. 2005-298379 discusses antiallergic effects of α-mangosteen and γ-mangosteen purified from mangosteen extract and effects of suppressing human pollinosis exhibited by α-mangosteen and γ-mangosteen. In these publications, however, effects on atopic dermatitis have not been examined. An object of the present invention is to provide a prophylactic and/or therapeutic agent for atopic dermatitis extracted from a mangosteen peel.

The present inventors conducted various researches to obtain a prophylactic and/or therapeutic agent for atopic dermatitis with minimal concerns of adverse drug reactions and high safety. As a result, they found that an agent extracted from a mangosteen peel with a polar solvent prevented or healed atopic dermatitis, and thus accomplished the present invention.

That is, the present invention provides a prophylactic and/or therapeutic agent for atopic dermatitis comprising an extract extracted from a mangosteen peel with a polar solvent. Furthermore, the present invention provides a food containing the above-mentioned prophylactic and/or therapeutic agent for atopic dermatitis.

The prophylactic and/or therapeutic agent for atopic dermatitis comprising an extract extracted from a mangosteen peel with a polar solvent of the present invention may be used in pets, livestock and/or poultry, in particular, dogs and/or cats. Furthermore, the present invention provides a feed containing a prophylactic and/or therapeutic agent for atopic dermatitis comprising an extract extracted from a mangosteen peel with a polar solvent.

Mangosteen peels can be obtained from a mangosteen fruit (fresh or dried product) and used. Mangosteen peels can be used as they are, but, in view of improvement of the extraction rate, it is preferable to crush or powder the peels before extraction. Furthermore, mangosteen peels can also be defatted with a non-polar solvent before extraction.

Extraction is performed using at least one polar solvent selected from the group consisting of methanol, ethanol, n-propanol, 2-propanol, n-butanol, acetone, ethyl acetate, and water, which are polar solvents. Two or more solvents can be used in combination. Taking into account the use as an orally-administered agent, a food, a drink, or a feed, ethanol or a combination of water and ethanol is preferably used as an extraction solvent in view of safety. The extraction temperature is not particularly specified, but a range from room temperature to the boiling point of a solvent is preferred in view of the extraction efficiency. The extraction time depends on the type of a solvent, the peel condition (fresh or dried product, crushed or powdered product, etc.), and the extraction temperature and is preferably in the range from 0.5 to 24 h.

If necessary, an extract may be concentrated or an extraction solvent may be removed using an evaporator. Furthermore, an extract can be used after being purified by solvent fractionation or chromatography, if necessary.

The prophylactic and/or therapeutic agent for atopic dermatitis of the present invention can be added to foods such as soft drinks, sweets, frozen desserts, dairy products, alcoholic beverages, and meats, and feeds.

The dose of a mangosteen peel extract as a prophylactic and/or therapeutic agent for atopic dermatitis varies depending on the administration method and the required treatment and cannot be specified categorically. However, the dose of an extract is 60 to 250 mg/kg in an animal or 0.3 to 300 mg/kg body weight/day in a human, more preferably 0.5 to 200 mg/kg body weight/day.

The mixed amount of the prophylactic and/or therapeutic food for atopic dermatitis of the present invention can be specified to satisfy the above-mentioned effective amount as a usual daily food intake. The daily amount can be divided into several times and ingested.

Furthermore, the mixed amount of the prophylactic and/or therapeutic feed for atopic dermatitis of the present invention can be specified to satisfy the above-mentioned effective amount as a usual daily feed intake. The daily amount can be divided into several times and ingested.

According to the present invention, atopic dermatitis can be prevented or healed using a mangosteen peel extract. Furthermore, the extract of the present invention is extracted from mangosteen with a polar solvent, and the above-mentioned effects can be obtained without performing further purification. When the mangosteen peel extract of the present invention is administered, effects comparable with or superior to those in the Protopic ointment applied group can be observed. Administration of the mangosteen peel extract of the present invention suppresses the increase in total blood IgE levels, indicating that allergic reactions are systemically suppressed.

Furthermore, mangosteen is called Queen of Fruit and the fruit of mangosteen is eaten. Mangosteen is a material widely recognized with a good image. Therefore, the prophylactic and/or therapeutic agent for atopic dermatitis comprising a mangosteen peel extract is easily accepted by patients without having concerns due to reputations. Furthermore, a feed containing the prophylactic and/or therapeutic agent for atopic dermatitis comprising a mangosteen peel extract is also easily accepted when it is used in livestock raised for food, and is easily accepted by pet lovers when it is used for pets such as dogs and cats.

### BRIEF DESCRIPTION OF THE DRAWING

FIG. 1 illustrates changes in clinical symptom scores over time in each group;
FIG. 2 illustrates scratching behavior frequencies before and after the start of the study in each group;
FIG. 3 illustrates scratching behavior durations before and after the start of the study in each group;
FIG. 4 illustrates blood IgE levels before and after the start of the study in each group;
FIG. 5 illustrates changes in transepidermal water loss over time in each group; and
FIG. 6 illustrates changes in body weights over time in each group.

### BEST MODES FOR CARRYING OUT THE INVENTION

Hereafter, the present invention will be described with reference to the following examples. However, the scope of the present invention is not limited to these examples.

### Example 1

A mangosteen peel extract was obtained as follows. Specifically, 100 g of an undried mangosteen peel was crushed and extracted in 11 of 70% ethanol with stirring at 80°C for 1 h. The extract was filtered, and the filtrate was dried under reduced pressure using an evaporator to obtain 27.4 g of an extract.

### Example 2

Effects of ingestion of a mangosteen peel extract on improvement of allergic dermatitis symptoms were verified using spontaneous atopic dermatitis model mice. For this experiment, 5- or 6-week-old NC/NgaTnd mice, which are spontaneous atopic dermatitis model mice, were used. To perform the experiment, these laboratory animals were subjected to one-week preliminary breeding before the start of the experiment and then assigned to any of three groups (n = 7 per group), a mangosteen peel extract fed group, a control feed fed group, and a Protopic ointment applied group. Statistical analyses were performed by Dunnett's multiple comparison test or Student's t test.

The mangosteen peel extract obtained in Example 1 was mixed in a feed (CRF-1, Oriental Yeast Co., Ltd.) at 0.25% (w/w) to obtain a mangosteen peel extract powder mixed feed. This feed was spontaneously fed to the mangosteen peel extract fed NC/NgaTnd mice, so that the intake of a mangosteen extract should be 250 mg/kg/day. NC/NgaTnd mice in the control feed fed group were spontaneously given a usual feed (CRF-1). NC/NgaTnd mice in the Protopic ointment applied group were spontaneously given a usual feed (CRF-1) as with the control feed fed group, and Protopic (0.1% ointment) was applied once daily five times weekly. Animals in any group were fed and given water ad libitum.

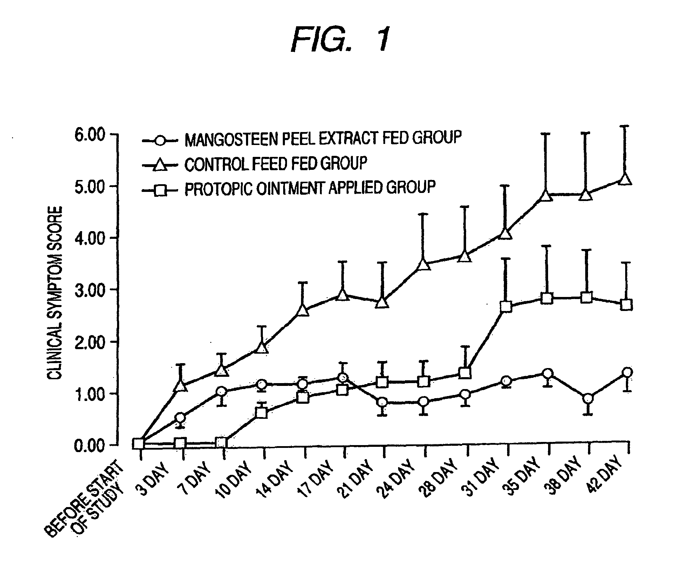

The above-mentioned three groups were evaluated for dermatitis scores. Evaluation was made as follows with reference to Matsuda, H. et al., Int. Immunol., 9: 461-466, 1997. Specifically, on the day before the start of feeding the study feed and twice weekly from the feeding start day to the day following the final feeding, five items of " itch" "erythema/hemorrhage," "edema," " excoriation/erosion," and " scaling/dryness" were evaluated using four ranks, "0 none," "1 mild," "2 moderate," and "3 severe." Different persons evaluated and fed animals throughout the study period, so that the person who performed evaluation would not find which animals belonged to which groups. The results are shown in Table 1 and FIG. 1. In FIG. 1, open circle denotes the mangosteen peel extract fed group, open triangle denotes the control feed fed group, and open square denotes the Protopic ointment applied group.

**Table 1 Mean clinical symptom scores and standard error in each group**

| | | | | | | | | | | | | | |
|---|---|---|---|---|---|---|---|---|---|---|---|---|---|
| (A, mangosteen peel extract fed group [n = 8]; B, control feed fed group [n = 7]; C, Protopic ointment applied group [n = 7]) | | | | | | | | | | | | | |

| Measurement day | Befor e start of study | Day 3 | Day 7 | Day 10 | Day 14 | Day 17 | Day 21 | Day 24 | Day 28 | Day 31 | Day 35 | Day 38 | Day 42 |
|---|---|---|---|---|---|---|---|---|---|---|---|---|---|
| Mean | | | | | | | | | | | | | |
| A | 0.00 | 0.50 | 1.00 | | 1.13 | 1.25 | 0.75 | 0.75 | 0.88 | 1.13 | 1.25 | 0.75 | 1.26 |
| B | 0.00 | 1.14 | 1.43 | 1.86 | 2.57 | 2.86 | 2.71 | 3.43 | 3.57 | 4.00 | 4.71 | 4.71 | 5.00 |
| C | 0.00 | 0.00 | 0.00 | 0.57 | 0.86 | 1.00 | 1.14 | 1.14 | 1.29 | 2.57 | 2.71 | 2.71 | 2.57 |
| | | | | | | | | | | P < 0.05 | | P < 0.01 | |
| Standard error | | | | | | | | | | | | | |
| A | 0.00 | 0.19 | 0.27 | 0.13 | 0.13 | 0.16 | 0.26 | 0.25 | 0.23 | 0.13 | 0.25 | 0.31 | 0.37 |
| B | 0.00 | 0.40 | 0.30 | 0.40 | 0.53 | 0.63 | 0.75 | 0.95 | 0.95 | 0.9 | 1.17 | 1.17 | 1.02 |
| C | 0.00 | 0.00 | 0.00 | 0.20 | 0.40 | 0.53 | 0.40 | 0.40 | 0.52 | 0.92 | 1.02 | 0.92 | 0.81 |

The clinical symptom score at the start of the study was 0 (no symptom developed) in all the groups. In the control feed fed group, clinical symptom scores increased with time from three days after the start of the study, and the clinical symptom scores at the end of the study were 5.0 ± 1.0. In the Protopic ointment applied group, mild dermatitis began to develop at 14 days after the start of the study, and the clinical symptom scores at the end of the study were 2.6 ± 0.8. In the mangosteen peel extract fed group, very mild dermatitis symptoms were observed from three days after the start of the study, but no marked exacerbation was observed. The clinical symptom scores at the end of the study were still as low as 1.3 ± 0.4. In the Protopic ointment applied group, significantly lower clinical symptom scores were maintained from Day 3 to Day 28 of the study as compared with the control feed fed group, but no statistically significant difference was observed from Day 31 after the start of the study. On the other hand, in the mangosteen peel extract fed group, a statistically significant difference was observed in suppression of clinical symptom scores from Day 14 after the start of the study to the end of the study. Throughout the study period, no remarkable adverse drug reaction due to the mangosteen peel extract feeding was observed.

### Example 3

Using three groups similar to the above-mentioned groups, pruritus frequency and duration were measured to verify effects of ingestion of the mangosteen peel extract on improvement of allergic dermatitis symptom. Measurement was performed as follows with reference to Orito, K. et al., Brit. J. Dermatol., 150: 1-6 (2004). To acclimatize mice in the measurement environment, mice in all the groups were acclimatized in a scratching frequency measurement system (SCLABA, Noveltec Inc.) for 20 min once daily for two days from three days before the start of feeding the study feed. The scratching frequencies and durations before the start of the study were measured by video recording for 20 min after 20-min acclimatization on the day before the start of feeding. After acclimatization before completion of feeding of the study feed in the same manner as described above, scratching frequencies and durations were recorded by videotaping on the day following the end of the study. Videotaping was performed between 12:00 to 18:00, and the date and the individual numbers were recorded.

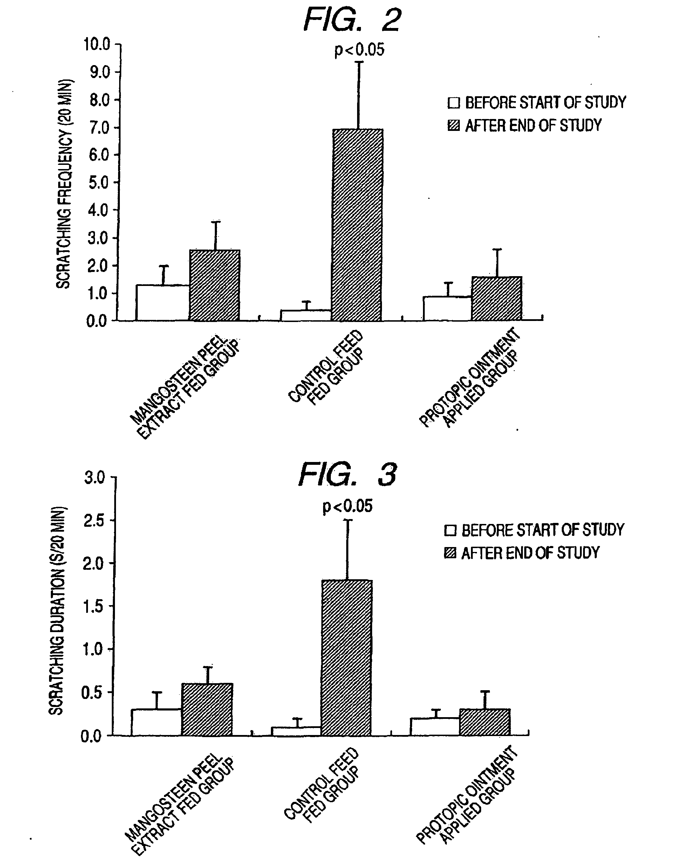

The results are shown in FIGS. 2 and 3. In all the groups, the scratching frequency was once, and the scratching duration (s) was approx. 0.2 s during 20 min of videotaping at the start of the study. In all the groups, scratching behaviors tended to increase after the end of the study (the day following the completion of the study feed feeding) as compared with those before the start of the study (day before the study feed feeding), but no statistically significant difference was observed in the mangosteen peel extract fed group or the Protopic ointment applied group. On the other hand, in the control feed fed group, marked increases were observed in the scratching behavior frequency at the end of the study as compared with that before the start of the study.

### Example 4

Using three groups similar to the above-mentioned groups, blood IgE concentrations were measured to verify effects of ingestion of the mangosteen peel extract on improvement of allergic dermatitis symptoms. The IgE concentrations were measured as follows with reference to Tanaka, A. et al., J. Invest. Dermatol., 127: 855-863 (2007). Approx. 1 ml of blood was collected under ether anesthesia using a heparin-treated syringe from the caudal vein before the start of feeding and from the abdominal aorta after video recording of the scratching frequencies and durations on the day of completion of feeding. The collected blood was subjected to centrifugation at 4°C to obtain plasma, and the plasma was cryopreserved at -20°C. IgE levels were measured by sandwich ELISA using anti-mouse IgE antibodies that recognize two different epitopes (ME-01-DE and ME-02-B, both produced by Yamasa Corporation).

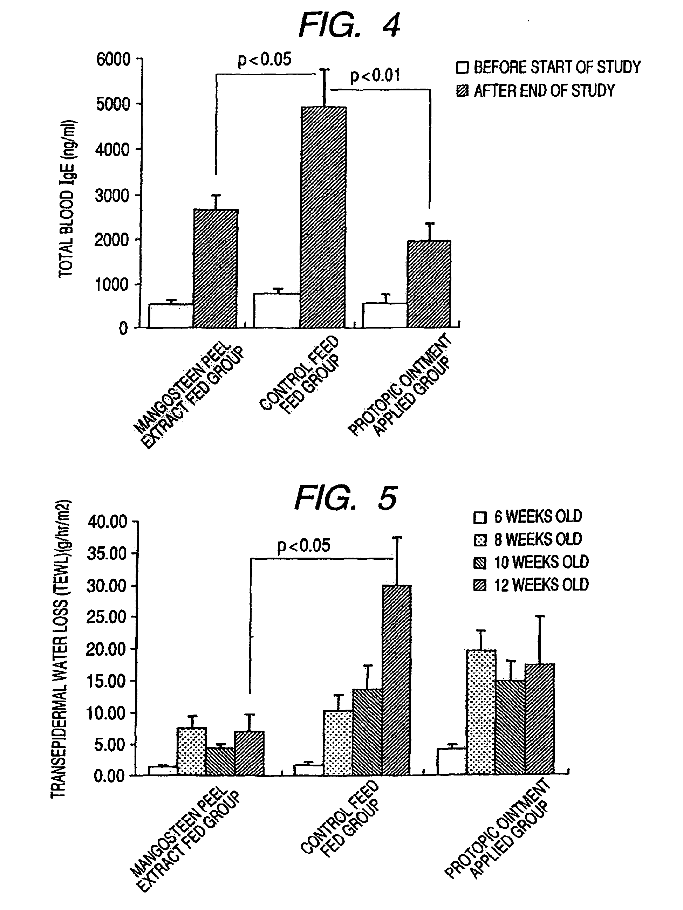

The results are shown in FIG. 4. The total blood IgE (ng/ml) before the start of the study was 531 ± 104 in the mangosteen peel extract fed group, 782 ± 108 in the control feed fed group, and 556 ± 207 in the Protopic ointment applied group, and no statistically significant difference was observed between the groups. In all the groups, the total blood IgE levels tended to increase after completion of the study as compared with those before the start of the study. However, a statistically significant suppression of increases in the total blood IgE levels was observed in the mangosteen peel extract fed group and the Protopic ointment applied group as compared with the control feed fed group.

### Example 5

Using three groups similar to the above-mentioned groups, transepidermal water loss (TEWL) was measured to verify effects of ingestion of the mangosteen peel extract on improvement of allergic dermatitis symptoms. The back of the mouse was shaved on the day before the measurement day, and TEWL was measured on the back using a multi-probe adaptor (CK Electronic). TEWL was measured three times at a time for each individual animal, and the mean thereof was regarded as the TEWL value. Measurement was performed twice, before and after the study, and once fortnightly therebetween.

The results are shown in FIG. 5. In all the groups, TEWL at the start of the study was in the normal range, with 5 g/h/m². TEWL tended to increase from two weeks after the start of the study. In particular, TEWL increased with time in the control feed fed group, with as high values as 29.7 ± 7.6 g/h/m² at the end of the study. TEWL also increased in the Protopic ointment applied group until four weeks after the start of the study, but was 17.1 ± 7.6 g/h/m² at the end of the study, which was lower than in the control feed fed group, although no statistically significant difference was observed. On the other hand, in the mangosteen peel extract fed group, TEWL remained in the normal range, with lower than 10 g/h/m², throughout the study period, and significantly lower values were observed at the end of the study as compared with the control feed fed group.

### Example 6

Body weight was measured in three groups similar to the above-mentioned groups. Using an electronic scale (HL-320, Ernst Hansen), body weight was measured fortnightly from the day before the start of the study feed feeding.

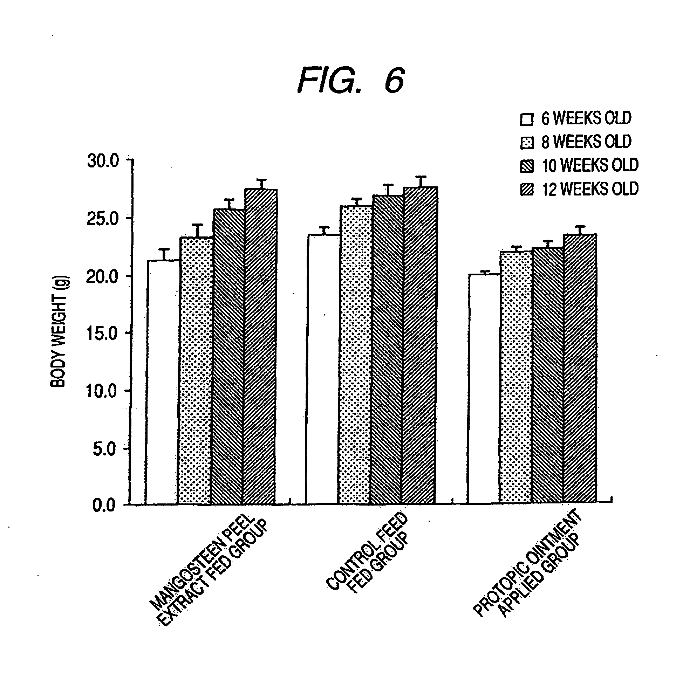

The results are shown in FIG. 6. The body weights at the start of the study were 20.0 to 23.6 g. The body weight increased with time in all the groups, and no difference was observed in the percentage body weight gain between the groups. No adverse drug reaction was observed after administration of mangosteen.

### Example 7

Using the plant extract prepared in Example 1, a lozenge was prepared as follows.

| | |
|---|---|
| Glucose | 72.3% |
| Lactose | 18.0 |
| Gum arabic | 6.0 |
| Flavor | 1.0 |
| Sodium monofluorophosphate | 0.7 |
| Mangosteen peel extract | 2.0 |
| | 100.0% |

### Example 8

Using the plant extract prepared in Example 1, a chewing gum was prepared as follows.

| | |
|---|---|
| Gum base | 20.0% |
| Sucrose | 54.7 |
| Glucose | 14.5 |
| Starch syrup | 9.3 |
| Flavor | 0.5 |
| Mangosteen peel extract | 1.0 |
| | 100.0% |

### Example 9

Using the plant extract prepared in Example 1, a candy was prepared as follows.

| | |
|---|---|
| Sucrose | 50.0% |
| Starch syrup | 33.4 |
| Citric acid | 1.0 |
| Flavor | 0.2 |
| Mangosteen peel extract | 1.0 |
| Water | 14.4 |
| | 100.0% |

### Example 10

Using the plant extract prepared in Example 1, a gummy sweet jelly was prepared as follows.

| | |
|---|---|
| Gelatin | 60.0% |
| Starch syrup | 23.0 |
| Sucrose | 7.5 |
| Plant oils and fats | 4.5 |
| Mannitol | 2.9 |
| Lemon juice | 1.0 |
| Mangosteen peel extract | 1.0 |
| | 100.0% |

### Example 11

Using the plant extract prepared in Example 1, a chocolate was prepared as follows.

| | |
|---|---|
| Powdered sugar | 40.8% |
| Cocoa bitter | 20.0 |
| Powdered whole milk | 20.0 |
| Cocoa butter | 17.0 |
| Mannitol | 1.0 |
| Mangosteen peel extract | 1.0 |
| Flavor | 0.2 |
| | 100.0% |

### Example 12

Using the plant extract prepared in Example 1, a sherbet was prepared as follows.

| | |
|---|---|
| Orange juice | 25.0% |
| Sucrose | 25.0 |
| Egg white | 10.0 |
| Mangosteen peel extract | 0.1 |
| Flavor | 0.1 |
| Water | 39.8 |
| | 100.0% |

### Example 13

Using the plant extract prepared in Example 1, an ice cream was prepared as follows.

| | |
|---|---|
| Powdered skim milk | 50.0% |
| Fresh cream | 25.0 |
| Sucrose | 10.0 |
| Egg yolk | 10.0 |
| Mangosteen peel extract | 1.0 |
| Flavor | 0.1 |
| Water | 3.9 |
| | 100.0% |

### Example 14

Using the plant extract prepared in Example 1, a biscuit was prepared as follows.

| | |
|---|---|
| First-class weak flour | 25.0% |
| First-class all-purpose flour | 22.0 |
| White sugar | 5.0 |
| Sodium chloride | 1.0 |
| Glucose | 1.0 |
| Palm shortening | 12.0 |
| Sodium hydrogencarbonate | 0.2 |
| Sodium bisulfite | 0.2 |
| Rice powder | 2.0 |
| Powdered whole milk | 1.0 |
| Powdered milk substitute | 0.6 |
| Mangosteen peel extract | 1.0 |
| Water | 29.0 |
| | 100.0% |

### Example 15

Using the plant extract prepared in Example 1, a confectionary tablet was prepared as follows.

| | |
|---|---|
| Sucrose | 75.8% |
| Glucose | 19.0 |
| Sucrose fatty acid ester | 0.2 |
| Flavor | 0.2 |
| Mangosteen peel extract | 0.8 |
| Water | 4.0 |
| | 100.0% |

### Example 16

Using the plant extract prepared in Example 1, a drink was prepared as follows.

| | |
|---|---|
| Orange juice | 30.0 |
| Isomerized sugar | 15.14 |
| Citric acid | 0.1 |
| Vitamin C | 0.04 |
| Flavor | 0.1 |
| Mangosteen peel extract | 0.2 |
| Water | 54.42 |
| | 100.0% |

### Example 17

Using the plant extract prepared in Example 1, a dog feed was prepared as follows.

| | |
|---|---|
| Meat material (chicken, beef) | 40.3 |
| Pork backfat | 2.5 |
| Ice | 19.0 |
| Plant proteins | 30.3 |
| Bone meal | 3.8 |
| Sodium chloride | 0.4 |
| Polymeric phosphate | 0.2 |
| Sugars/seasoning | 2.5 |
| Mangosteen peel extract | 1.0 |
| | 100.0% |

### Example 18

Using the plant extract prepared in Example 1, a cat feed was prepared as follows.

| | |
|---|---|
| Corn | 30.0 |
| Wheat flour | 35.0 |
| Fish meal | 15.0 |
| Meat meal | 8.9 |
| Beef fat | 4.0 |
| Sodium chloride | 1.0 |
| Bonito extract | 5.0 |
| Vitamins | 0.4 |
| Minerals | 0.4 |
| Mangosteen peel extract | 0.3 |
| | 100.0% |

### Example 19

Using the plant extract prepared in Example 1, a swine feed was prepared as follows.

| | |
|---|---|
| Corn | 65.0 |
| Milo | 5.0 |
| Defatted rice bran | 3.0 |
| Corn germ meal | 2.0 |
| Soybean cake | 17.0 |
| Fish meal | 4.0 |
| Animal oils and fats | 2.0 |
| Calcium carbonate | 1.0 |
| Premix | 0.9 |
| Mangosteen peel extract | 0.1 |
| | 100.0% |

This application claims priority from Japanese Patent Application No. 2008-134246 filed May 22, 2008, which is hereby incorporated by reference herein.

## Claims

1. A prophylactic and/or therapeutic agent for atopic dermatitis comprising an extract extracted from a mangosteen (Garcinia mangostana L.) peel with a polar solvent.

2. The prophylactic and/or therapeutic agent for atopic dermatitis according to claim 1, wherein the polar solvent is ethanol or aqueous ethanol.

3. A prophylactic and/or therapeutic agent for atopic dermatitis, **characterized by** containing an extract extracted from a mangosteen (Garcinia mangostana L.) peel with ethanol or aqueous ethanol as an active ingredient, which suppresses IgE antibody production.

4. A food containing the prophylactic and/or therapeutic agent for atopic dermatitis according to any one of claims 1 to 3.

5. A food for prophylactic and/or therapeutic treatment of pruritus containing an extract of mangosteen (Garcinia mangostana L.) peel as an active ingredient, wherein the extract is extracted with ethanol or an aqueous ethanol.

6. A feed containing the prophylactic and/or therapeutic agent for atopic dermatitis according to any one of claims 1 to 3.

7. A feed for prophylactic and/or therapeutic treatment of pruritus containing an extract of mangosteen (Garcinia mangostana L.) peel as an active ingredient, wherein the extract is extracted with ethanol or an aqueous ethanol.
